# FASCICULE DE BREVET EUROPEEN

(11) **EP 3 589 227 B1**
(45) Date de publication et mention de la délivrance du brevet: **24.02.2021**
(21) Numéro de dépôt: 18712954.9
(22) Date de dépôt: 05.03.2018
(51) Int. Cl.: A61B 90/00, A61C 9/00, A61C 19/045

(54) **DISPOSITIF DE FIXATION MANDIBULAIRE D'UN MARQUEUR DE LOCALISATION**
VORRICHTUNG ZUR MANDIBULÄREN BEFESTIGUNG EINES ORTSMARKERS
DEVICE FOR MANDIBULAR ATTACHMENT OF A LOCATION MARKER

(30) Priorité: 03.03.2017 FR 1751748
(43) Date de publication de la demande: 08.01.2020
(73) Titulaire: Modjaw, 69100 Villeurbanne (FR)
(72) Inventeur: JAISSON, Maxime, 73800 Les Marches (FR)
(74) Mandataire: Regimbeau
(86) Numéro de dépôt international: PCT/FR2018/050498
(87) Numéro de publication internationale: WO 2018/158551

(56) Documents cités:
- DE-A1-102014 111 643
- DE-U1-202015 105 356
- US-A- 4 432 728
- US-A1- 2006 201 520
- US-A1- 2016 317 108

## Description

### DOMAINE DE L'INVENTION

La présente invention concerne un dispositif de fixation mandibulaire d'un marqueur de localisation, destiné notamment à être utilisé pour l'enregistrement de la cinématique mandibulaire d'un individu.

### ETAT DE LA TECHNIQUE

Le document WO 2013/030511 décrit un procédé de conception d'un appareil dentaire pour un individu dans lequel on utilise la cinématique mandibulaire enregistrée sur ledit individu pour animer des modèles numériques des arcades dentaires supérieure et inférieure de l'individu.

Pour ce faire, il est tout d'abord nécessaire de recaler les modèles numériques des arcades à des plans et points de référence de l'individu.

Ensuite, on enregistre la cinématique mandibulaire de l'individu. A cet effet, on équipe l'individu de plusieurs marqueurs :
- un ou plusieurs marqueurs sont positionnés au niveau du front de l'individu, par l'intermédiaire d'un support ceignant le front,
- un ou plusieurs marqueurs sont positionnés au niveau de la mâchoire inférieure (mandibule) et solidarisés aux dents de l'arcade inférieure.

Une caméra adaptée pour détecter lesdits marqueurs est orientée vers le visage du patient et enregistre les déplacements des marqueurs au cours de mouvements de la mandibule.

Les marqueurs mandibulaires peuvent être fixés directement sur les dents de l'arcade inférieure, mais ceci nécessite l'utilisation d'un dispositif d'écartement des lèvres pour les rendre visibles par la caméra.

Une autre solution est de fixer le marqueur à un support intermédiaire qui est lui-même fixé à la mandibule. Ledit support intermédiaire doit être fixé d'une manière rigide à la mandibule, car tout déplacement relatif par rapport à la mandibule pendant l'acquisition de la cinématique mandibulaire détériorerait la précision de l'enregistrement. De plus, le port de ce support intermédiaire doit être confortable pour l'individu.

Le marqueur est de préférence fourni séparément du support, pour permettre d'abord la mise en place du support seul dans la bouche du patient, puis, une fois que le support est fixé sur la mandibule, le marqueur est monté sur le support. Cependant, si l'effort de mise en place du marqueur sur le support est trop élevé, le support est susceptible de se déplacer par rapport à la mandibule.

Les documents DE 10 2014 111643 A1 et DE 20 2015 105356 U1 décrivent tous deux un système de connexion par encliquetage avec ergots et logements correspondants, entre un marqueur extra-oral et son dispositif de fixation sur la mâchoire, en particulier la mandibule, d'un patient.

Un but de l'invention est donc de concevoir un support pour un marqueur mandibulaire de localisation qui permette une fixation rigide du marqueur par rapport à l'individu, avec un système de fixation du marqueur qui minimise l'effort de montage du marqueur sur le support.

A cet effet, l'invention propose un dispositif de localisation de la mâchoire inférieure d'un individu, comprenant :
(a) un marqueur comprenant une face intérieure pourvue de deux ergots de fixation, et
(b) un dispositif de fixation dudit marqueur à la mâchoire inférieure de l'individu, comprenant :
   - une partie intrabuccale présentant une forme générale de U adaptée pour venir en contact avec la face externe des dents de la mâchoire inférieure,
   - une partie extrabuccale comprenant un élément de fixation pour le marqueur,
   - une partie de liaison raccordant la partie intrabuccale et la partie extrabuccale,
l'élément de fixation comprenant deux logements adaptés pour recevoir chacun un ergot respectif du marqueur, lesdits logements étant séparés par une languette adaptée pour se déformer élastiquement lorsque l'un des ergots est engagé dans un logement respectif,
le marqueur étant fixé à la partie extrabuccale du dispositif de fixation par engagement de chaque ergot dans un logement respectif, la languette déformée élastiquement exerçant un effort de pression sur l'un des ergots.

Selon un mode de réalisation, l'élément de fixation comprend en outre deux ailettes latérales adaptées pour se déformer élastiquement lorsque chaque ergot est engagé dans un logement respectif de sorte à exercer un effort de pression sur la face intérieure du marqueur.

De manière particulièrement avantageuse, la partie intrabuccale est flexible dans le plan du U.

Selon un mode de réalisation préféré, la face de la partie intrabuccale orientée vers les dents présente une surface rugueuse.

De manière avantageuse, la face de la partie intrabuccale orientée vers les dents comprend au moins une encoche adaptée pour s'engager dans un relief des dents de la mâchoire inférieure.

De préférence, la partie intrabuccale est pourvue à ses extrémités de segments sécables..

Selon un mode de réalisation, l'élément de fixation comprend des moyens de guidage et/ou de butée du marqueur.

De manière préférée, l'élément de fixation est configuré pour permettre un démontage du marqueur.

De manière avantageuse, la partie de liaison est conçue pour que, lorsque le dispositif est en place dans la bouche de l'individu, une section de ladite partie de liaison s'étende sensiblement dans le plan de fermeture des lèvres.

Selon un mode de réalisation, la partie intrabuccale, la partie extrabuccale et la partie de liaison sont formées d'un seul tenant.

La partie intrabuccale, la partie extrabuccale et la partie de liaison sont avantageusement constituées d'un matériau biocompatible.

Selon un mode de réalisation, le marqueur comprend une pluralité de pastilles réfléchissantes.

L'invention concerne également un système de localisation comprenant une caméra infrarouge et un ensemble formé du dispositif de fixation et du marqueur tel que décrit précédemment.

Enfin, l'invention concerne un procédé d'enregistrement de la cinématique mandibulaire d'un individu au moyen du système de localisation tel que décrit précédemment, dans lequel on fixe le marqueur sur les dents de l'arcade mandibulaire de l'individu par l'intermédiaire du dispositif de fixation, on fixe un autre marqueur muni d'une pluralité de pastilles réfléchissantes sur le front de l'individu, et l'on enregistre avec la caméra infrarouge les déplacements relatifs desdits marqueurs lors de mouvements mandibulaires réalisés par l'individu.

Pour fixer le marqueur sur le dispositif de fixation, on présente la face intérieure du marqueur pourvue des deux ergots de fixation en regard de la partie extrabuccale du dispositif, on engage un premier ergot dans un premier logement puis un second ergot dans un second logement tout en déformant élastiquement la languette de sorte que ladite languette exerce un effort de pression sur le second ergot.

### DESCRIPTION DES FIGURES

D'autres caractéristiques et avantages de l'invention ressortiront de la description détaillée qui va suivre, en référence aux dessins annexés sur lesquels :
- la figure 1 est une vue en perspective du dispositif de fixation mandibulaire équipé d'un marqueur de localisation,
- la figure 2 est une vue en perspective du dispositif de fixation mandibulaire de la figure 1 en l'absence du marqueur de localisation,
- la figure 3 est une vue de face du dispositif de fixation mandibulaire de la figure 2,
- la figure 4 est une vue de côté du dispositif de fixation mandibulaire de la figure 2,
- la figure 5 est une vue de dessus du dispositif de fixation mandibulaire de la figure 2,
- la figure 6 est une vue de côté du marqueur mandibulaire.

### DESCRIPTION DETAILLEE DE L'INVENTION

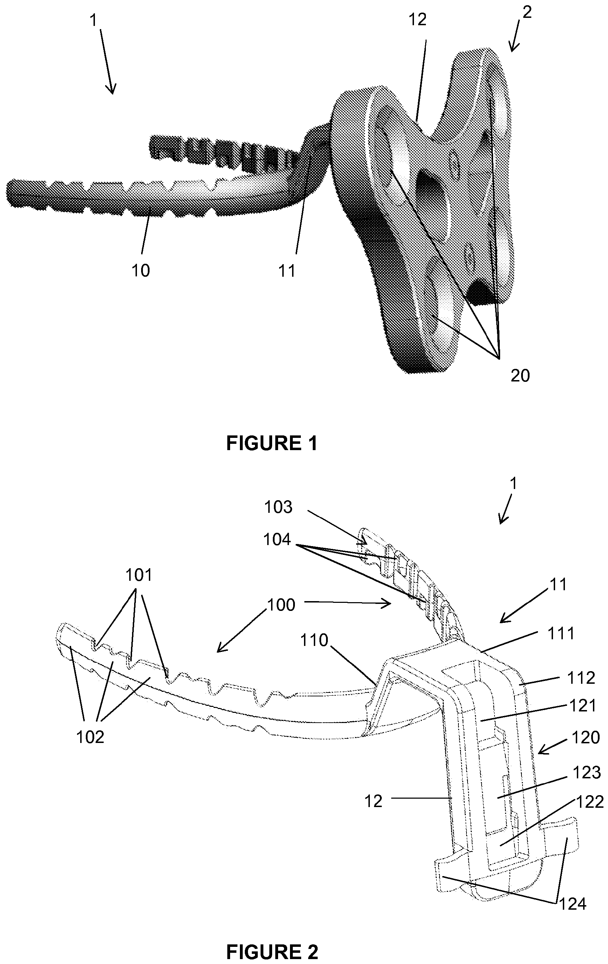

La figure 1 est une vue en perspective d'un marqueur 2 monté sur un dispositif 1 de fixation mandibulaire selon l'invention.

La technologie de localisation du marqueur n'est pas limitative.

Dans l'exemple illustré sur la figure 1, le marqueur comprend quatre pastilles réfléchissantes 20, dont la taille et la position relative sont connues, détectables par une caméra infrarouge. La forme des pastilles n'est pas limitative. Ainsi, lesdites pastilles pourraient être remplacées par des éléments réfléchissants présentant toute autre forme appropriée, par exemple des billes.

De manière alternative, les pastilles réfléchissantes pourraient être remplacées par une mire formée d'un motif bicolore de géométrie connue.

Selon d'autres modes de réalisation, les pastilles réfléchissantes pourraient être remplacées par des diodes, des bobines électromagnétiques, ou tout autre moyen de localisation approprié, tel que des capteurs inertiels (IMU, acronyme du terme anglo-saxon « inertial measurement unit »), des accéléromètres, des gyroscopes, etc.

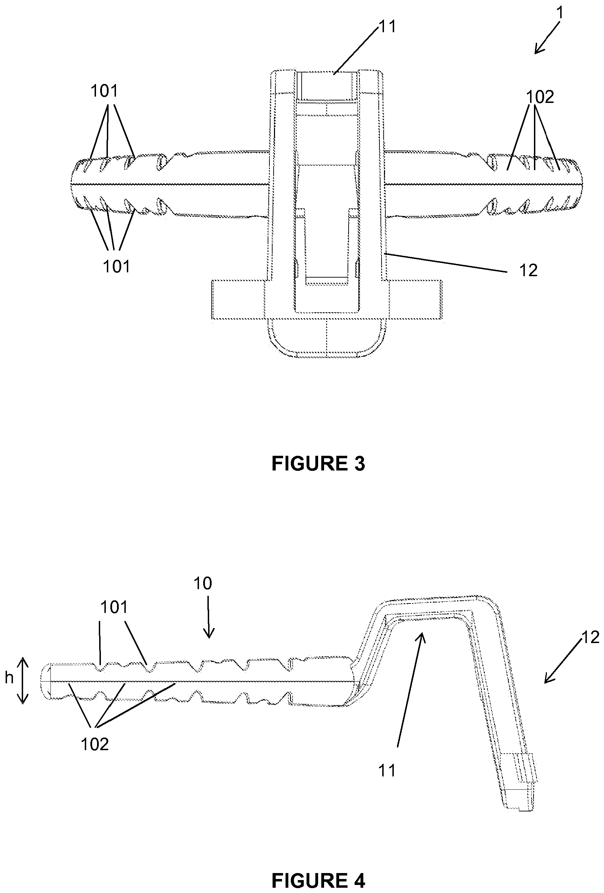

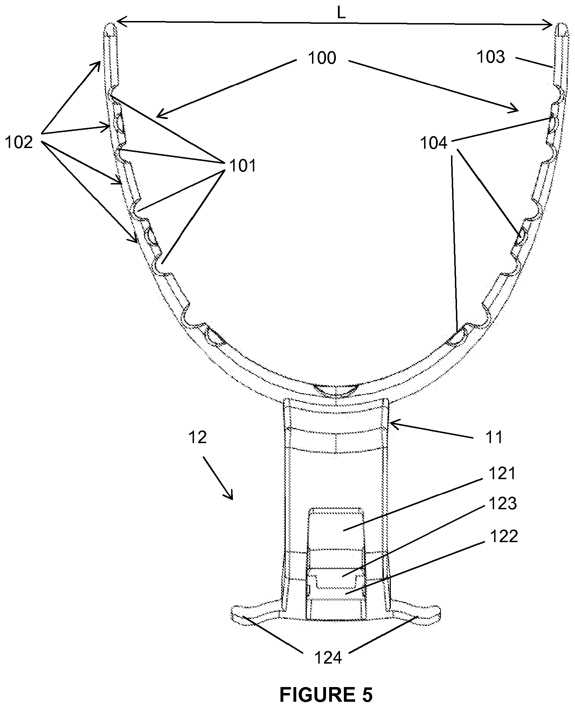

Comme illustré sur la figure 1, le dispositif 1 comprend :
- une partie intrabuccale 10 destinée à être maintenue contre la face externe des dents de la mâchoire inférieure (non représentées),
- une partie extrabuccale 12 (mieux visibles sur les figures 2 à 5) comprenant un élément de fixation pour le marqueur 2,
- une partie de liaison 11 permettant de raccorder la partie intrabuccale 10 à la partie extrabuccale 12.

De manière particulièrement avantageuse, l'ensemble des trois parties 10, 11, 12 est formé d'un seul tenant, par moulage d'un matériau thermoplastique biocompatible.

Par exemple, ce matériau peut être du polyamide, du polypropylène, du PEEK (liste non exhaustive).

Le dispositif 1 est typiquement à usage unique et peut ainsi être jeté après utilisation.

En vue de son utilisation, et selon les éventuelles exigences réglementaires, le dispositif de fixation mandibulaire peut être fourni stérilisé dans une enveloppe étanche le protégeant de toute contamination extérieure.

Les figures 2 à 5 illustrent des vues du dispositif de fixation mandibulaire sans le marqueur.

La partie intrabuccale 10 présente une forme générale de U.

Le matériau et l'épaisseur de la partie intrabuccale sont choisis pour présenter une certaine flexibilité dans un sens d'écartement ou de rapprochement des jambes du U dans leur plan.

Ainsi, les deux jambes 100 peuvent être écartées par rapport à leur position initiale pour être insérées dans la bouche de l'individu sans frotter contre les dents ou les gencives, puis relâchées une fois qu'elles ont été correctement positionnées.

Au repos, c'est-à-dire avant insertion dans la bouche de l'individu, la largeur L de la partie intrabuccale est définie comme étant la distance maximale entre les deux jambes du U.

De préférence, la largeur de la partie intrabuccale au repos est légèrement inférieure à la largeur moyenne de la mâchoire de l'individu (définie comme étant la distance entre les surfaces extérieures des molaires de la mâchoire inférieure), de sorte qu'une fois insérée dans la bouche du patient, l'élasticité des jambes 100 exerce un léger effort de compression sur les dents, afin d'assurer une bonne tenue de la partie intrabuccale.

Selon un mode de réalisation optionnel mais avantageux, l'extrémité des jambes du U est rendue sécable par la présence d'une ou plusieurs encoches 101 qui définissent un ou plusieurs segments détachables 102. Ainsi, on peut retirer un ou plusieurs desdits segments détachables jusqu'à obtenir une longueur des jambes 100 adaptée à la longueur de la mâchoire de l'individu. La longueur desdits segments 102 (distance entre deux encoches 101 adjacentes) est typiquement de l'ordre de 5 à 10 mm. Les encoches 101 peuvent s'étendre à partir de la face inférieure ou de la face supérieure des jambes 100 ; elles peuvent également s'étendre à partir des deux faces en vis-à-vis les unes des autres, comme c'est le cas sur les figures 1 à 5. La profondeur des encoches 101 (ou la somme des profondeurs dans le cas des encoches en vis-à-vis) est typiquement de l'ordre de la moitié de la hauteur h de chaque jambe.

De manière avantageuse, la tenue mécanique de la partie intrabuccale 10 sur la mâchoire du patient est assurée au moins en partie par une colle biocompatible (non représentée) déposée entre la surface des dents et la face intérieure de la partie intrabuccale. Une telle colle biocompatible est fréquemment utilisée dans le domaine de la dentisterie.

Par ailleurs, la face intérieure 103 de la partie intrabuccale, c'est-à-dire la face destinée à venir en contact avec les dents, peut présenter une certaine rugosité. Par exemple, une pièce issue d'impression 3D (technique de frittage laser dite SLS, acronyme du terme anglo-saxon « Selective Laser Sintering ») présente une rugosité appropriée. Dans le cas d'une pièce issue de moulage, le moule d'injection peut subir un traitement, par exemple un sablage ou un gravage chimique, lui conférant un état de surface non lisse. Cette rugosité permet d'améliorer la tenue mécanique de la colle vis-à-vis de la face intérieure 103.

D'autre part, la face intérieure 103 de la partie intrabuccale peut être pourvue d'encoches 104. Lesdites encoches sont agencées de manière à se trouver en regard de parties en relief sur la surface externe des dents lorsque le dispositif est en place dans la bouche de l'individu. Les encoches 104 remplissent donc une fonction de blocage de la partie intrabuccale, notamment dans la direction antéro-postérieure, qui stabilisent le dispositif dans la bouche.

De manière particulièrement avantageuse, la hauteur h de la partie intrabuccale est choisie suffisamment faible (typiquement inférieure à la hauteur moyenne des dents) afin de ne pas dépasser du plan formé par la surface supérieure des dents de la mâchoire inférieure lorsque le dispositif est en place dans la bouche de l'individu. Par exemple, la hauteur h est de l'ordre de 5 mm.

Ainsi, la partie intrabuccale n'interfère pas avec la cinématique mandibulaire (serrement des dents, mouvements de mastication et autres).

La partie extrabuccale 12 présente quant à elle un élément de fixation 120 pour un marqueur.

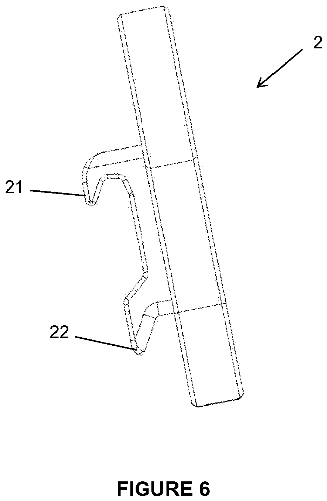

Dans l'exemple illustré sur les figures annexées, le marqueur 2 est fixé par encliquetage sur la partie extrabuccale 12. A cet effet, comme illustré sur la figure 6, le marqueur mandibulaire 2 comprend, sur sa face intérieure (i.e. opposée aux pastilles réfléchissantes), deux ergots 21, 22 agencés l'un au-dessus de l'autre (en considérant la position du marqueur lorsqu'il est en place sur l'individu). L'élément de fixation 120 comprend deux logements 121, 122 séparés par une languette élastique 123. Pour mettre en place le marqueur 2 sur le dispositif de fixation 1, l'ergot supérieur 21 est tout d'abord engagé dans le logement 121, puis l'ergot inférieur est engagé dans le logement 122, cet engagement entraînant une légère déformation de la languette 123 vers l'arrière (c'est-à-dire du côté opposé aux pastilles réfléchissantes). Le marqueur est donc maintenu sur la partie extrabuccale 12 par engagement des ergots 21, 22 dans les logements 121, 122 et par un effort de pression exercé par la languette 123 déformée.

L'assemblage du marqueur 2 sur le dispositif de fixation est avantageusement effectué une fois que le dispositif de fixation a été mis en place dans la bouche du patient et fixé à l'aide de la colle biocompatible susmentionnée. Ainsi, le marqueur n'entrave pas cette opération de mise en place.

Grâce au mécanisme de fixation décrit ci-dessus, le marqueur peut être manipulé d'une seule main par le praticien pour être mis en place sur la partie extrabuccale du dispositif.

Par ailleurs, l'effort nécessaire pour la mise en place du marqueur sur la partie extrabuccale est très faible, de sorte qu'il n'affecte pas la tenue mécanique du dispositif de fixation vis-à-vis de la mandibule.

De manière particulièrement avantageuse, l'élément de fixation 120 comprend en outre deux ailettes élastiques latérales 124, qui se déforment lors de la mise en place du marqueur mandibulaire sur la partie extrabuccale 12. Lesdites ailettes exercent alors un effort de pression sur la face intérieure du marqueur, ce qui stabilise encore sa fixation.

On notera que la mise en place du marqueur est particulièrement facilitée lorsque les ergots et logements sont agencés l'un au-dessus de l'autre dans la position d'utilisation du dispositif, car le logement supérieur est aisément accessible au praticien. Cependant, une architecture similaire avec un alignement des logements et de la languette dans un plan horizontal, ou selon toute autre inclinaison, pourrait être également être mise en œuvre sans pour autant sortir du cadre de la présente invention. Par exemple, la partie extrabuccale pourrait comprendre un logement droit et un logement gauche alignés dans un plan horizontal et, pour l'assemblage, le marqueur devrait être présenté devant la partie extrabuccale avec les ergots alignés selon un plan horizontal, afin de permettre l'engagement des ergots dans les logements et la déformation de la languette dans un plan horizontal.

La partie extrabuccale est conçue pour être suffisamment rigide pour supporter le poids dudit marqueur sans se déformer.

De préférence, le mode de fixation du marqueur 2 sur la partie extrabuccale 12 est choisi pour ne pas nécessiter d'outil. Par exemple, la partie extrabuccale peut présenter un élément permettant l'encliquetage du marqueur, une fixation de type quart de tour, voire par force magnétique, etc. Dans ce dernier cas, la partie extrabuccale et le marqueur sont respectivement pourvus d'un aimant et/ou d'un élément en matériau ferreux destiné à coopérer avec l'aimant.

De manière particulièrement avantageuse, le marqueur est démontable du dispositif de fixation.

Selon un mode de réalisation, la fixation pour le marqueur est en outre reproductible, c'est-à-dire qu'à chaque fois que l'on met en place le marqueur sur le dispositif, la position du marqueur par rapport au dispositif est identique.

A cet effet, l'élément de fixation 120 prévu sur la partie extrabuccale 12 comprend des moyens de guidage et/ou de butée pour le marqueur.

Selon un mode de réalisation moins préféré, le marqueur pourrait former partie intégrante de la partie extrabuccale.

Lorsque le dispositif 1 est en place dans la bouche de l'individu, la partie extrabuccale 12 est avantageusement située en-dessous du plan des lèvres de l'individu (le marqueur 20 pouvant quant à lui dépasser de ce plan).

Pour des raisons de stabilité du dispositif, celui-ci est sensiblement symétrique par rapport au plan antéro-postérieur de l'individu.

La partie 11 de liaison entre les parties intrabuccale 10 et extrabuccale 12 se présente comme un pontet s'étendant à partir de la région centrale de la partie intrabuccale 10. Ce pontet présente une première section 110 s'étendant vers la mâchoire supérieure, une deuxième section 111 s'étendant au travers des lèvres et une troisième section 112 de raccordement à la partie extrabuccale 12.

La forme et les dimensions de la partie de liaison sont choisies pour que, lorsque le dispositif est en place dans la bouche de l'individu, la deuxième section 111 s'étende au niveau du plan des lèves, sans exercer de pression sur les lèvres. Ainsi, le dispositif 1 reste immobile même en cas de déglutition.

Par ailleurs, la partie de liaison 11 est conçue pour être suffisamment rigide pour ne pas se déformer lorsque le marqueur 20 est fixé à la partie extrabuccale 12.

Le dispositif 1 et le marqueur 2 font partie d'un système de localisation dans lequel ils sont associés à une caméra, notamment une caméra infrarouge dans le cas d'un marqueur muni de pastilles réfléchissantes.

Pour l'enregistrement de la cinématique mandibulaire d'un individu, la partie intrabuccale est fixée au contact des dents de l'arcade inférieure de l'individu, puis le marqueur est attaché à la partie extrabuccale.

L'individu est également équipé d'un support frontal portant également un marqueur de localisation selon la même technologie que le marqueur mandibulaire. Eventuellement, on peut s'affranchir d'un tel support frontal en équipant la mâchoire supérieure d'un dispositif de fixation similaire à celui qui vient d'être décrit, avec des formes et dimensions adaptées à cette mâchoire.

La mise en place de ces marqueurs sur l'individu est naturellement réalisée de manière non invasive.

La caméra est placée en vis-à-vis du patient, de sorte que le marqueur frontal et le marqueur mandibulaire soient en permanence dans son champ de vision.

L'individu exécute alors un certain nombre de mouvements mandibulaires (mastication, etc.) selon les préconisations du praticien.

La caméra détecte et enregistre les déplacements relatifs des marqueurs.

Après l'acquisition, cet enregistrement est appliqué aux modèles numériques des arcades dentaires de l'individu, ce qui permet de les animer.

## Revendications

1. Dispositif de localisation de la mâchoire inférieure d'un individu, comprenant :
(a) un marqueur (2) comprenant une face intérieure pourvue de deux ergots (21, 22) de fixation, et
(b) un dispositif (1) de fixation dudit marqueur (2) à la mâchoire inférieure de l'individu, comprenant :
- une partie intrabuccale (10) présentant une forme générale de U adaptée pour venir en contact avec la face externe des dents de la mâchoire inférieure,
- une partie extrabuccale (12) comprenant un élément (120) de fixation pour le marqueur (2),
- une partie de liaison (11) raccordant la partie intrabuccale et la partie extrabuccale,
l'élément (120) de fixation comprenant deux logements (121, 122) adaptés pour recevoir chacun un ergot (21, 22) respectif du marqueur (2), lesdits logements (121, 122) étant séparés par une languette (123) adaptée pour se déformer élastiquement lorsque l'un (22) des ergots est engagé dans un logement (122) respectif, le marqueur (2) étant fixé à la partie extrabuccale (12) du dispositif de fixation par engagement de chaque ergot (21, 22) dans un logement (121, 122) respectif, la languette (123) déformée élastiquement exerçant un effort de pression sur l'un (22) des ergots.

2. Dispositif selon la revendication 1, dans lequel l'élément de fixation (120) comprend en outre deux ailettes latérales (124) adaptées pour se déformer élastiquement lorsque chaque ergot (21, 22) est engagé dans un logement (121, 122) respectif de sorte à exercer un effort de pression sur la face intérieure du marqueur (2).

3. Dispositif selon l'une des revendications 1 ou 2, dans lequel la partie intrabuccale (10) est flexible dans le plan du U.

4. Dispositif selon l'une des revendications 1 à 3, dans lequel la face (103) de la partie intrabuccale (10) orientée vers les dents présente une surface rugueuse.

5. Dispositif selon l'une des revendications 1 à 4, dans lequel la face (103) de la partie intrabuccale (10) orientée vers les dents comprend au moins une encoche (104) adaptée pour s'engager dans un relief des dents de la mâchoire inférieure.

6. Dispositif selon l'une des revendications 1 à 5, dans lequel la partie intrabuccale (10) est pourvue à ses extrémités de segments sécables (102).

7. Dispositif selon l'une des revendications 1 à 6, dans lequel l'élément de fixation (120) comprend des moyens de guidage et/ou de butée du marqueur (2).

8. Dispositif selon l'une des revendications 1 à 7, dans lequel l'élément de fixation (120) est configuré pour permettre un démontage du marqueur (2).

9. Dispositif selon l'une des revendications 1 à 8, dans lequel la forme et les dimensions de la partie de liaison (11) sont choisies pour que, lorsque le dispositif de fixation (1) est en place dans la bouche de l'individu, une section (111) de ladite partie de liaison s'étende sensiblement dans le plan de fermeture des lèvres.

10. Dispositif selon l'une des revendications 1 à 9, dans lequel la partie intrabuccale (10), la partie extrabuccale (12) et la partie de liaison (11) sont formées d'un seul tenant.

11. Dispositif selon l'une des revendications 1 à 10, dans lequel la partie intrabuccale (10), la partie extrabuccale (12) et la partie de liaison (11) sont constituées d'un matériau biocompatible.

12. Dispositif selon l'une des revendications 1 à 11, dans laquelle le marqueur (2) comprend une pluralité de pastilles (20) réfléchissantes.

13. Système de localisation comprenant une caméra et un dispositif de localisation selon l'une des revendications 1 à 12.2, notamment une caméra infrarouge dans le cas d'un marqueur muni de pastilles réfléchissantes.

14. Procédé d'enregistrement de la cinématique mandibulaire d'un individu au moyen du système de localisation selon la revendication 13, dans lequel on fixe le marqueur (2) sur les dents de l'arcade mandibulaire de l'individu par l'intermédiaire du dispositif de fixation (1), on fixe un autre marqueur muni d'une pluralité de pastilles réfléchissantes sur le front de l'individu, et l'on enregistre avec la caméra infrarouge les déplacements relatifs desdits marqueurs lors de mouvements mandibulaires réalisés par l'individu.

15. Procédé selon la revendication 14, dans lequel, pour fixer le marqueur (2) sur le dispositif de fixation (1), on présente la face intérieure du marqueur pourvue des deux ergots (21,22) de fixation en regard de la partie extrabuccale du dispositif, on engage un premier ergot (21) dans un premier logement (121) puis un second ergot (22) dans un second logement (122) tout en déformant élastiquement la languette (123) de sorte que ladite languette (123) exerce un effort de pression sur le second ergot (22).

## Patentansprüche

1. Vorrichtung zur Ortung des Unterkiefers eines Individuums, Folgendes umfassend:
(a) einen Marker (2) umfassend eine Innenseite, die mit zwei Nasen (21, 22) zur Befestigung versehen ist, und
(b) eine Vorrichtung (1) zur Befestigung des Markers (2) am Unterkiefer des Individuums, Folgendes umfassend:
- einen intraoralen Teil (10), der eine allgemeine U-Form aufweist, angepasst, um in Kontakt mit der Außenseite der Zähne des Unterkiefers zu kommen,
- einen extraoralen Teil (12), der ein Element (120) zur Befestigung für den Marker (2) umfasst,
- einen Verbindungsteil (11), der den intraoralen Teil und den extraoralen Teil anschließt, wobei das Element (120) zur Befestigung zwei Aufnahmen (121, 122) umfasst, die angepasst sind, um jede eine jeweilige Nase (21, 22) des Markers (2) zu empfangen, wobei die Aufnahmen (121, 122) durch eine Lasche (123) getrennt sind, die angepasst ist, um sich elastisch zu verformen, wenn eine (22) der Nasen in eine jeweilige Aufnahme (122) eingeführt ist,
wobei der Marker (2) an dem extraoralen Teil (12) der Befestigungsvorrichtung durch Einführen jeder Nase (21, 22) in eine jeweilige Aufnahme (121, 122) befestigt ist, wobei die elastisch verformte Lasche (123) eine Druckkraft auf eine (22) der Nasen ausübt.

2. Vorrichtung nach Anspruch 1, wobei das Befestigungselement (120) weiter zwei Seitenflügel (124) umfasst, die angepasst sind, um sich elastisch zu verformen, wenn jede Nase (21, 22) in eine jeweilige Aufnahme (121, 122) eingeführt ist, um eine Druckkraft auf die Innenseite des Markers (2) auszuüben.

3. Vorrichtung nach einem der Ansprüche 1 oder 2, wobei der intraorale Teil (10) in der Ebene des U flexibel ist.

4. Vorrichtung nach einem der Ansprüche 1 bis 3, wobei die zu den Zähnen gerichtete Seite (103) des intraoralen Teils (10) eine raue Oberfläche aufweist.

5. Vorrichtung nach einem der Ansprüche 1 bis 4, wobei die zu den Zähnen gerichtete Seite (103) des intraoralen Teils (10) mindestens eine Kerbe (104) umfasst, die angepasst ist, um in ein Relief der Zähne des Unterkiefers einzugreifen.

6. Vorrichtung nach einem der Ansprüche 1 bis 5, wobei der intraorale Teil (10) an seinen Enden mit teilbaren Segmenten (102) versehen ist.

7. Vorrichtung nach einem der Ansprüche 1 bis 6, wobei das Befestigungselement (120) Führungs- und/oder Anschlagmittel des Markers (2) umfasst.

8. Vorrichtung nach einem der Ansprüche 1 bis 7, wobei das Befestigungselement (120) konfiguriert ist, um eine Demontage des Markers (2) zu erlauben.

9. Vorrichtung nach einem der Ansprüche 1 bis 8, wobei die Form und die Abmessungen des Verbindungsteils (11) ausgewählt sind, damit, wenn die Befestigungsvorrichtung (1) im Mund des Individuums vorhanden ist, sich eine Sektion (111) des Verbindungsteils im Wesentlichen in der Schließebene der Lippen erstreckt.

10. Vorrichtung nach einem der Ansprüche 1 bis 9, wobei der intraorale Teil (10), der extraorale Teil (12) und der Verbindungsteil (11) aus einem Stück gebildet sind.

11. Vorrichtung nach einem der Ansprüche 1 bis 10, wobei der intraorale Teil (10), der extraorale Teil (12) und der Verbindungsteil (11) aus einem biokompatiblen Material konstituiert sind.

12. Vorrichtung nach einem der Ansprüche 1 bis 11, wobei der Marker (2) eine Vielzahl von reflektierenden Pastillen (20) umfasst.

13. System zur Ortung, umfassend eine Kamera und eine Vorrichtung zur Ortung nach einem der Ansprüche 1 bis 12, insbesondere eine Infrarotkamera im Falle eines Markers, der mit reflektierenden Pastillen versehen ist.

14. Verfahren zum Aufzeichnen der Unterkiefer-Kinematik eines Individuums anhand des Systems zur Ortung nach Anspruch 13, wobei der Marker (2) an den Zähnen des Unterkieferbogens des Individuums anhand der Befestigungsvorrichtung (1) befestigt wird, ein weiterer Marker, der mit einer Vielzahl von reflektierenden Pastillen versehen ist, an der Stirn des Individuums befestigt wird, und mit der Infrarotkamera die relativen Verschiebungen der Marker bei Unterkieferbewegungen, die von dem Individuum realisiert werden, aufgezeichnet werden.

15. Verfahren nach Anspruch 14, wobei zum Befestigen des Markers (2) an der Befestigungsvorrichtung (1) die Innenseite des Markers, die mit den beiden Nasen (21, 22) zum Befestigen versehen ist, auf Höhe des extraoralen Teils der Vorrichtung angesetzt wird, eine erste Nase (21) in eine erste Aufnahme (121) eingeführt wird, danach eine zweite Nase (22) in eine zweite Aufnahme (122) eingeführt wird, und dabei die Lasche (123) derart elastisch verformt, dass die Lasche (123) eine Druckkraft auf die zweite Nase (22) ausübt.

## Claims

1. Device for localization of the lower jaw of an individual, comprising:
(a) a marker (2) comprising an inner face provided with two attachment lugs (21, 22), and
(b) a device (1) for attaching said marker (2) to the lower jaw of the individual, comprising:
- an intra-oral portion (10) having a general U shape adapted for coming into contact with the outer face of the teeth of the lower jaw,
- an extra-oral portion (12) comprising an attachment element (120) for the marker (2),
- a connecting portion (11) connecting the intra-oral portion and the extra-oral portion,
the attachment element (120) comprising two recesses (121, 122) each adapted for receiving a respective lug (21, 22) of the marker (2), said recesses (121, 122) being separated by a tab (123) adapted for being elastically deformed when one (22) of the lugs is engaged in a respective recess (122),
the marker (2) being attached to the extra-oral portion (12) of the attachment device by engagement of each lug (21, 22) in a respective recess (121, 122), the elastically deformed tab (123) exerting a pressure force on one (22) of the lugs.

2. Device according to claim 1, wherein the attachment element (120) further comprises two lateral fins (124) adapted for deforming elastically when each lug (21, 22) is engaged in a respective recess (121, 122) so as to exert a pressure force on the inner face of the marker (2).

3. Device according to one of claims 1 or 2, wherein the intra-oral portion (10) is flexible in the plane of the U.

4. Device according to one of claims 1 to 3, wherein the face (103) of the intra-oral portion (10) oriented towards the teeth has a rough surface.

5. Device according to one of claims 1 to 4, wherein the face (103) of the intra-oral portion (10) oriented towards the teeth comprises at least one notch (104) adapted for engaging in a relief of the teeth of the lower jaw.

6. Device according to one of claims 1 to 5, wherein the intra-oral portion (10) is provided at its ends with divisible segments (102).

7. Device according to one of claims 1 to 6, wherein the attachment element (120) comprises means for guiding and/or abutting the marker (2).

8. Device according to one of claims 1 to 7, wherein the attachment element (120) is configured to enable dismantling of the marker (2).

9. Device according to one of claims 1 to 8, wherein the shape and the dimensions of the connecting portion (11) are chosen so that, when the attachment device (1) is in place in the mouth of the individual, a section (111) of said connecting portion extends substantially in the plane of the closing of the lips.

10. Device according to one of claims 1 to 9, wherein the intra-oral portion (10), the extra-oral portion (12) and the connecting portion (11) are integrally formed.

11. Device according to one of claims 1 to 10, wherein the intra-oral portion (10), the extra-oral portion (12) and the connecting portion (11) are formed from a biocompatible material.

12. Device according to one of claims 1 to 11, wherein the marker (2) comprises a plurality of reflective patches (20).

13. Localization system comprising a camera and a localization device according to one of claims 1 to 12, in particular an infrared camera in the case of a marker provided with reflective patches.

14. Method for recording the mandibular kinematics of an individual by means of the localization system according to claim 13, wherein the marker (2) is attached on the teeth of the mandibular arch of the individual through the attachment device (1), another marker provided with a plurality of reflective patches is attached on the forehead of the individual, and the relative displacements of said markers during mandibular movements made by the individual are recorded with the infrared camera.

15. Method according to claim 14, wherein, to attach the marker (2) on the attachment device (1), the inner face of the marker provided with two attachment lugs (21,22) is presented facing the extra-oral portion of the device, a first lug (21) is engaged in a first recess (121) then a second lug (22) in a second recess (122) while elastically deforming the tab (123) so that said tab (123) exerts a pressure force on the second lug (22) .
